# DEMANDE DE BREVET EUROPEEN

(11) **EP 2 072 024 A1**
(43) Date de publication de la demande: **24.06.2009**
(21) Numéro de dépôt: 07301689.1
(22) Date de dépôt: 18.12.2007
(51) Int. Cl.: A61F 2/14

(54) **Insertion d'un objet décoratif ou d'un marquage dans la cornée d'un oeil**

(71) Demandeur: Telandro, Alain, 06400 Cannes (FR)
(72) Inventeur: Telandro, Alain, 06400 Cannes (FR)
(74) Mandataire: de Beaumont, Michel

(57) **Abrégé**

L'invention concerne un procédé et système d'insertion d'au moins un objet décoratif ou d'un marquage dans la cornée (1) d'un oeil, comportant les étapes suivantes : réaliser, par laser et hors de l'aire pupillaire, au moins une cavité intra cornéenne (10) débouchant à la surface de l'oeil par au moins une fente (12) ou canal ; et introduire un matériau formant l'objet décoratif ou le marquage dans la cavité par la fente ou canal.

## Description

### Domaine de l'invention

La présente invention concerne de façon générale les accessoires d'ornements utilisés par l'être humain afin de modifier son apparence dans un but esthétique ou de marquage.

### Exposé de l'art antérieur

Les techniques utilisées pour associer au corps des objets décoratifs, par exemple des bijoux, incluent des techniques invasives pour fixer ces objets de façon durable (réversible ou non). Par exemple, la technique dite de piercing consiste à fixer, par l'intermédiaire d'un canal dans l'épiderme, un objet décoratif (anneau, clou, etc.). Une autre technique connue consiste à apporter des déformations à la surface du corps par l'insertion de corps étrangers sous la peau. De même, les techniques de tatouage consistent à insérer des encres sous la peau pour donner des motifs visibles.

Un organe qui se remarque particulièrement d'un point de vue esthétique est l'oeil, ne serait-ce que par les différences de couleur qu'il procure entre les individus. Modifier l'esthétique de l'oeil s'effectue aujourd'hui au moyen de lentilles cornéennes qui peuvent n'avoir aucune fonction de correction de la vision. Ces lentilles comportent, en regard de l'iris, une zone colorée avec ou sans motif afin de modifier l'aspect de l'oeil. De telles lentilles cornéennes sont des accessoires d'ornements non durables qui requièrent un entretien régulier (nettoyage, hydratation, etc.). De plus, les lentilles permettent difficilement de présenter des motifs ayant une orientation angulaire déterminée.

Il serait souhaitable de pouvoir associer à l'oeil un ornement, de façon plus durable qu'une lentille.

Il serait également souhaitable de pouvoir sélectionner une orientation angulaire de l'ornement.

Une difficulté réside dans le fait que la fonction de l'oeil qui constitue un organe particulièrement sensible du corps humain ne doit pas être perturbée.

Une autre difficulté est que l'oeil est un organe mobile qui se trouve périodiquement recouvert par les paupières, ce qui requiert de préserver une surface externe régulière.

### Résumé de l'invention

L'invention vise à orner l'oeil d'un objet décoratif ou d'un marquage.

Un autre objet vise à préserver la surface externe régulière de l'oeil.

Un autre objet vise à éviter de recouvrir cette surface externe par un corps étranger de type lentille.

Un autre objet vise une solution réversible.

Pour atteindre tout ou partie de ces objets ainsi que d'autres, il est prévu un procédé d'insertion d'au moins un objet décoratif ou d'un marquage dans la cornée d'un oeil, comportant les étapes suivante :
réaliser, par laser et hors de l'aire pupillaire, au moins une cavité intra cornéenne débouchant à la surface de l'oeil par au moins une fente ou canal ; et
introduire un matériau formant l'objet décoratif ou le marquage dans la cavité par la fente ou canal.

Selon un mode de mise en oeuvre de la présente invention, la cavité définit :
au moins un logement de réception du matériau de direction principale approximativement parallèle à la surface de la cornée, le logement débouchant par une fente dont l'aplomb sépare le logement en deux parties dans sa direction principale ; et
un espace de déformation, communiquant avec l'extrémité d'une desdites parties du logement, d'épaisseur inférieure à celle du logement et de longueur, dans ladite direction principale, au moins égale à celle de l'autre partie.

Selon un mode de mise en oeuvre de la présente invention, l'objet est introduit par la fente en direction de l'espace de déformation jusqu'à ce qu'il soit entièrement dans la cavité, puis ramené en direction de la deuxième partie du logement jusqu'à libérer l'espace de déformation.

Selon un mode de mise en oeuvre de la présente invention, l'objet est enrobé d'un matériau biocompatible avec la nature de la cornée préalablement à son insertion.

Selon un mode de mise en oeuvre de la présente invention, le matériau est une pierre.

Selon un mode de mise en oeuvre de la présente invention, le matériau est métallique.

Selon un mode de mise en oeuvre de la présente invention, le matériau est une matière plastique.

Selon un mode de mise en oeuvre de la présente invention, la cavité définit :
au moins un logement de réception du matériau de direction principale approximativement parallèle à la surface de la cornée, le logement débouchant par au moins un premier canal d'introduction d'un outil de dépôt du matériau à l'intérieur du logement, le canal étant obturable par une lèvre déformable, ménagée dans l'épaisseur de la cornée ; et
au moins un canal d'évent établissant une deuxième communication entre le logement et la surface de l'oeil.

Selon un mode de mise en oeuvre de la présente invention, le matériau est un gel ou liquide coloré, non susceptible de diffuser dans la cornée.

Il est également prévu un système d'insertion d'un objet décoratif ou d'un marquage dans la cornée d'un oeil, hors de l'aire pupillaire, comportant :
un laser programmé pour la réalisation d'une cavité ; et
un matériau formant un objet décoratif ou un marquage à loger dans la cavité.

### Brève description des dessins

Ces objets, caractéristiques et avantages, ainsi que d'autres seront exposés en détail dans la description suivante de modes de mise en oeuvre et de réalisation particuliers faite à titre non-limitatif en relation avec les figures jointes parmi lesquelles :
la figure 1 est une coupe transversale schématique d'un oeil ;
la figure 2 représente un détail de la coupe de la figure 1 illustrant un mode de mise en oeuvre de l'invention ;
la figure 3 est une vue de face correspondant à la figure 2 ;
les figures 4A, 4B, 4C et 4D illustrent, respectivement par une vue de dessus et par des coupes transversales partielles, un mode de mise en oeuvre de l'invention ;
les figures 5A, 5B et 5C sont des coupes transversales partielles illustrant un autre mode de mise en oeuvre ;
la figure 6 est une vue partielle illustrant différentes variantes de réalisation de cavités d'inclusion d'objets décoratifs ; et
la figure 7 illustre une autre variante d'une cavité.

De mêmes éléments ont été désignés par de mêmes références aux différentes figures.

### Description détaillée

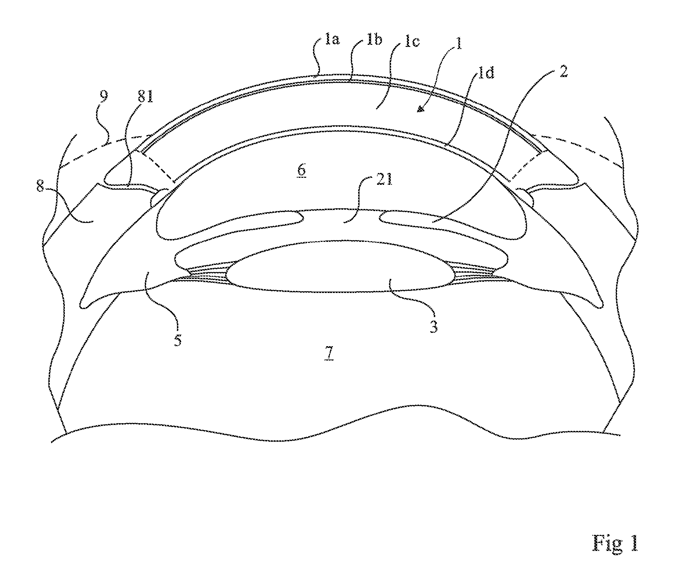

La figure 1 est une coupe transversale schématique et partielle d'un oeil faisant apparaître, à l'arrière de la cornée 1, l'iris 2 et le cristallin 3. La périphérie externe de l'iris 2 rejoint l'uvée 5 (le corps ciliaire) auquel est lié le cristallin 3. L'ensemble baigne dans l'humeur aqueuse 6 (entre la cornée et le cristallin) ou vitrée 7 (derrière le cristallin). En périphérie de la cornée, se trouve la sclère 8 recouverte à l'extérieur par la conjonctive 9 qui forme le blanc de l'oeil et qui s'arrête en périphérie de la cornée. Dans la représentation de la figure 1 apparaissent des canaux lacrymaux 81 entre la sclère et la cornée. La pupille 21, formée par l'ouverture centrale de l'iris 2 au diamètre variable contrôle le flux de lumière pénétrant dans l'oeil en traversant la cornée.

La cornée est constituée de différentes couches. A l'extérieur se trouve l'épithélium cornéen 1a qui présente une épaisseur d'environ 50 µm représentant approximativement un dixième de l'épaisseur totale de la cornée. En dessous se trouve la membrane de Bowman 1b qui sépare l'épithélium cornéen du stroma cornéen 1c. Le stroma 1c constitue la couche principale de la cornée. Son épaisseur est généralement comprise entre 400 et 600 µm. Côté intérieur de l'oeil, l'endothélium cornéen 1d donne à la cornée sa transparence.

Selon un mode de mise en oeuvre, l'invention prévoit de ménager une cavité dans la cornée afin d'y inclure un objet décoratif ou un marquage. Cette cavité est créée principalement dans le stroma cornéen et hors de l'aire pupillaire (hors du champ de vision), c'est-à-dire à l'aplomb d'une partie de l'iris qui subsiste quelle que soit la dilatation de la pupille.

La cavité est obtenue au moyen d'un laser du type de ceux utilisés habituellement à des fins curatives, par exemple des interventions de chirurgie pour inclure des "intacts" dans la cornée afin de modifier sa courbure et corriger ainsi des astigmatismes asymétriques (par exemple, le kératocône). Les "intacts" sont des segments arciformes de plexiglas qui sont glissés dans des espaces créés dans la cornée afin d'en modifier la courbure. L'invention prévoit d'utiliser ce type de laser hors du champ de vision afin de ne pas affecter la fonctionnalité de l'oeil. Un exemple de laser utilisable est un laser fournissant des impulsions d'une durée de l'ordre de la femtoseconde. Un tel outil est susceptible de réaliser des incisions dans la cornée avec une précision meilleure que 10 µm. Par exemple, des lasers connus sous la dénomination commerciale FEMTEC conviennent.

Par rapport à d'autres lasers, les lasers femtoseconde permettent de réaliser des cavités intra cornéennes sans qu'il soit nécessaire de découper une partie externe pour rendre accessible la partie à creuser. La précision d'un laser femtoseconde permet de ne vaporiser que des zones enterrées dans la cornée sans détruire les zones superficielles. Un tel laser peut être programmé pour réaliser la cavité en mode automatique.

La cavité créée au moyen du laser comporte au moins un espace ou logement destiné à recevoir au moins un matériau d'ornement formant un objet décoratif ou un marquage. Les formes et dimensions de ce logement sont donc adaptées aux dimensions de l'objet ou du marquage. Une fente ou canal reliant le logement à l'extérieur de l'oeil sert à introduire le matériau. La cavité est principalement réalisée dans le stroma cornéen dont l'épaisseur est compatible avec différentes tailles d'objets à implanter. En pratique, l'épaisseur maximale du logement ne devra pas dépasser 200 µm afin de laisser subsister au moins une épaisseur de l'ordre de 250 µm de stroma cornéen sous le logement. L'épaisseur de stroma au dessus du logement est par exemple d'au moins 100 µm.

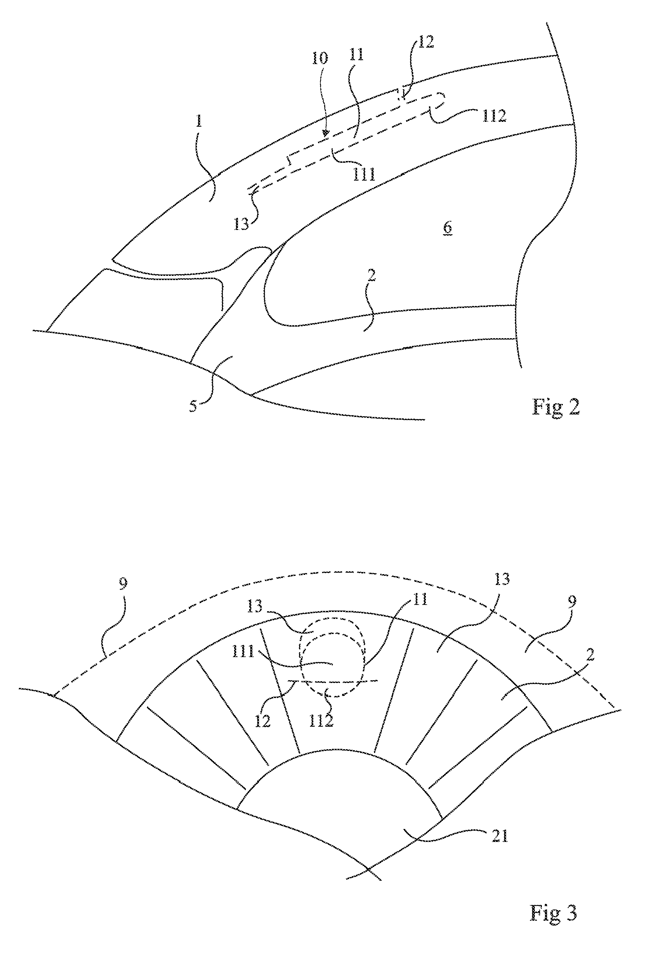

Les figures 2 et 3 sont respectivement des vues en coupe et de face d'une partie d'un oeil dans une zone ou a été réalisée une cavité intra cornéenne 10.

La cavité 10 comporte un espace ou logement 11 de réception d'un objet qui communique avec l'extérieur par une fente 12, de préférence rectiligne, servant à introduire l'objet. Cette fente 12 n'est pas réalisée à une extrémité du logement mais de façon que son aplomb sépare le logement 11 en deux parties 111 et 112. La direction approximativement perpendiculaire au plan de la fente 12 définit la direction principale (arbitrairement longitudinale) de la cavité 10. La longueur de la fente 12 est de préférence supérieure à la largeur de la cavité dans une proportion fonction de l'épaisseur de l'objet, de façon à autoriser une déformation non traumatisante permettant d'introduire l'objet. La cavité est complétée par un espace 13 de glissement ou de déformation servant lors de la mise en place de l'objet. Cet espace 13 communique avec la première partie 111 du logement, du côté de son extrémité opposée à la fente. L'espace 13 est d'épaisseur inférieure à celle du logement 11 et sa longueur, dans la direction principale de la cavité 10, correspond approximativement à celle de la deuxième partie 112 du logement.

Dans l'exemple de la figure 3, le logement 11 a une forme générale circulaire, par exemple celle d'un disque. Un laser femtoseconde permet toutefois de créer une cavité intra cornéenne de toute forme souhaitée (par exemple, sphérique, ovoïde, parallélépipédique, polygonale, en forme de disque, tubulaire, etc.).

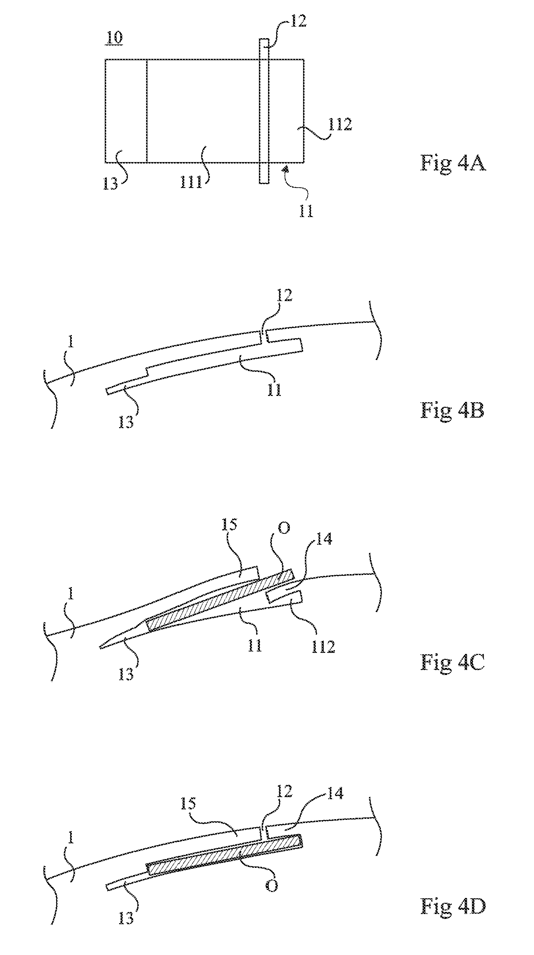

Les figures 4A, 4B, 4C, 4D illustrent un premier mode d'insertion d'un objet décoratif dans la cornée.

La figure 4A est une vue de face représentant uniquement le logement 10 (cavité 11 et espace 13) et la fente 12. Les figures 4B, 4C et 4D sont des vues partielles en coupe transversale de la zone de la cornée à différentes étapes du procédé d'insertion. Dans l'exemple représenté, on suppose un logement de forme générale parallélépipédique rectangle.

La figure 4B illustre la cavité vide, réalisée avec le procédé décrit ci-dessus, avant insertion de l'objet.

La figure 4C illustre la mise en place d'un objet décoratif 0. Celui-ci est glissé à partir de la fente 12 en direction de l'espace de glissement 13 en déformant, vers l'intérieur de l'oeil, la partie 14 de la cornée située au-dessus de la partie 112 du logement 11. Cette partie 14 forme en quelque sorte une lèvre se déformant vers le bas pour laisser passer l'objet O alors que la partie 15 de la cornée au-dessus de la partie 111 du logement 11 se déforme vers l'extérieur. L'objet O est inséré jusqu'à échapper la lèvre 14 en s'engageant dans l'espace de glissement 13. Puis (figure 4D), il est ramené sous la lèvre 14 après que celle-ci ait repris sa position normale. L'objet O occupe alors l'intégralité du logement 11. L'espace de glissement 13 ainsi que la fente 12 se referment.

La technique proposée ne requiert aucune suture de la cornée dans la mesure où la fente 12 se referme sans tension.

Différents types d'objets peuvent être insérés. Par exemple, il pourra s'agir de pastilles métalliques réfléchissantes, d'éléments plastiques colorés de formes diverses, de minéraux (pierres précieuses ou semi-précieuses), d'objets minces sous forme de films plastiques ou métalliques, de filaments, etc. Selon la nature de l'objet, il pourra être requis de l'enrober d'un matériau biocompatible préalablement à son insertion, par exemple du plexiglas.

En variante, plusieurs objets peuvent être introduits à partir d'une même fente dans une même cavité comportant plusieurs logements et espaces de glissement (par exemple, en étoile).

Selon une autre variante, une même cavité comporte plusieurs fentes.

Selon une autre variante, le ou les objets forment un marquage.

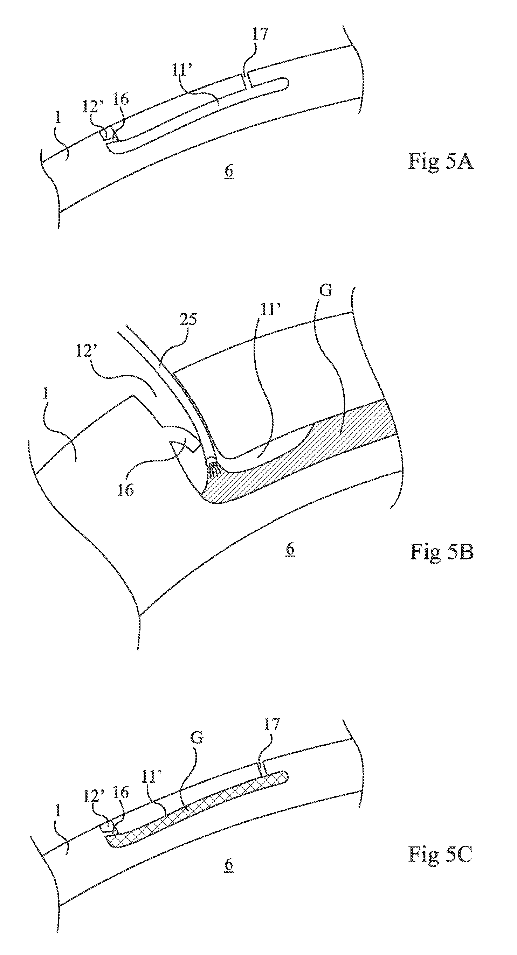

Les figures 5A, 5B et 5C illustrent un autre mode de mise en oeuvre dans lequel le logement 11' est utilisé pour recevoir un gel G, non susceptible de diffuser dans la cornée, constituant l'objet décoratif inséré ou un marquage. Le logement 11' est, par exemple, de forme générale tubulaire approximativement parallèle à la surface de la cornée. La fente 12 est remplacée par un canal de communication 12' permettant l'introduction d'une canule 25 d'injection du gel G. Une lèvre 16 formant clapet d'obturation du canal 12' est définie par le laser lors de la formation de la cavité. Cette lèvre 16 se déforme (figure 5B) lors de l'introduction de la canule pour reprendre sa forme d'origine une fois le gel introduit et la canule sortie (figure 5C), obturant ainsi le canal 12' pour retenir le gel G à l'intérieur du logement 11'.

Par rapport au mode de réalisation précédent, un espace de glissement n'est pas nécessaire. Par contre, un canal secondaire 17, fente ou espace de communication d'autre forme entre le logement 11' et l'extérieur de l'oeil est de préférence prévu et est disposé à proximité de l'autre extrémité du logement 11' par rapport au canal 12'. Ce canal formant évent permet d'évacuer l'air contenu dans le logement 11' lors de l'introduction du gel.

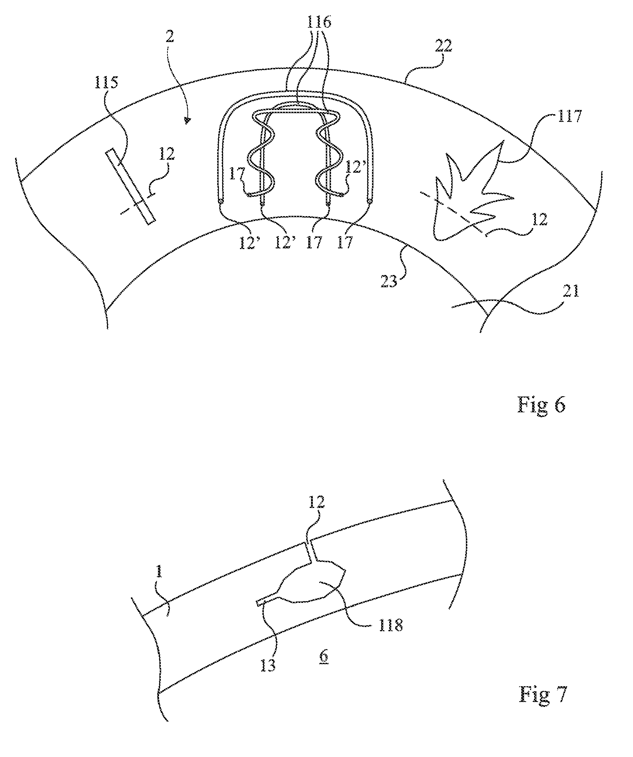

La figure 6 est une vue de dessus partielle d'un oeil illustrant d'autres exemples de mise en oeuvre. L'aplomb de l'iris hors de l'aire pupillaire est illustré par deux traits référencés 22 et 23.

Selon un premier exemple, un logement 115 est ménagé selon une direction radiale.

Selon un deuxième exemple, plusieurs logements tubulaires 116 de formes diverses pour effectuer un dessin sont réalisés dans la cornée. Chaque logement possède son propre canal 12' et évent 17. Les logements peuvent être côte à côte et/ou superposés. Par exemple, ils forment un marquage (inscription ayant une signification) d'un ou plusieurs symboles, lettres ou chiffres.

Selon un troisième exemple, un logement 117 a une forme générale plane reproduisant une feuille d'arbre. L'objet est alors par exemple un film coloré enroulé sur lui-même qui est introduit par la fente 12. Une fois dans le logement 117, le film se déplie et épouse la forme du logement 117.

La figure 7 est une vue en coupe illustrant une autre variante selon laquelle un logement 118 a une forme polygonale apte à recevoir une pierre taillée. Là encore, les dimensions du logement 118, de la fente 12 et de l'espace de glissement 13 sont adaptées à la pierre.

Quel que soit le mode de mise en oeuvre, la réalisation de la cavité 10 et la mise en place du matériau s'effectue hors de l'aire pupillaire et n'a aucune vertu curative ou réparatrice, mais uniquement pour objet d'adjoindre un ornement à l'oeil.

Un avantage de l'invention est qu'elle préserve l'aspect de surface de l'oeil et ne constitue aucune excroissance par rapport à la surface de la cornée. Elle est compatible avec le port de lentilles cornéennes, notamment correctrices.

Un autre avantage est que la technique proposée est durable et ne nécessite pas d'entretien contrairement à l'utilisation d'une lentille.

Un autre avantage est qu'elle permet d'effectuer des insertions localisées dont la position angulaire peut être choisie.

Un autre avantage est que la modification est réversible. Dans le cas d'un objet solide O, le corps étranger décoratif peut être explanté. Il suffit de faire sortir l'objet par sa fente d'introduction en réutilisant l'espace de glissement. En effet, la cavité intra cornéenne 11 et la fente 12, obtenues par vaporisation de la structure cornéenne au moyen du laser, ne se reconstituent pas. L'espace de glissement 13 reste donc utilisable pour extraire l'objet sans traumatisme. Dans le cas d'un gel G ou liquide coloré, celui-ci peut être retiré par lavage de la cavité en ouvrant la lèvre 16.

Il est donc possible de changer d'objets décoratifs (par exemple en remplaçant une pierre précieuse par une autre) en utilisant le même logement. On peut également redonner à l'oeil son aspect d'origine. Dans ce cas, une fois l'objet explanté, la cavité pourra être remplie d'un sérum physiologique ou, si le volume du logement est trop important, comblée par un implant transparent, par exemple en acide hyaluronique réticulé, compatible avec la nature de la cornée. De tels implants transparents sont déjà utilisés, par exemple, dans la chirurgie du glaucome.

Des modes de réalisation particuliers de la présente invention ont été décrits. Diverses variantes et modifications apparaîtront à l'homme de l'art. En particulier, le choix du matériau, le nombre de logements réalisés dans la cornée et leurs emplacements dépendent de l'effet souhaité. En outre, l'emploi d'un laser, son paramétrage ou programmation pour la mise en oeuvre de l'invention n'ont pas été détaillés, car ils sont à la portée d'un homme de l'art formé à l'utilisation d'un tel laser. En pratique, un anesthésiant local est utilisé pendant la mise en oeuvre du procédé d'insertion (réalisation de la cavité et mise en place de l'objet). Enfin, les différents modes de réalisation et variantes peuvent être combinés.

## Revendications

1. Procédé d'insertion d'au moins un objet décoratif ou d'un marquage dans la cornée (1) d'un oeil, comportant les étapes suivantes :
réaliser, par laser et hors de l'aire pupillaire, au moins une cavité intra cornéenne (10) débouchant à la surface de l'oeil par au moins une fente (12) ou canal (12') ; et
introduire un matériau formant l'objet décoratif (O, G) ou le marquage dans la cavité par la fente ou canal.

2. Procédé selon la revendication 1, dans lequel la cavité (10) définit :
au moins un logement (11, 115, 117, 118) de réception du matériau (O) de direction principale approximativement parallèle à la surface de la cornée (1), le logement débouchant par une fente (12) dont l'aplomb sépare le logement en deux parties (111, 112) dans sa direction principale ; et
un espace de déformation (13), communiquant avec l'extrémité d'une (111) desdites parties du logement, d'épaisseur inférieure à celle du logement et de longueur, dans ladite direction principale, au moins égale à celle de l'autre partie (112).

3. Procédé selon la revendication 2, dans lequel l'objet (O) est introduit par la fente (12) en direction de l'espace de déformation (13) jusqu'à ce qu'il soit entièrement dans la cavité (11, 115, 117, 118), puis ramené en direction de la deuxième partie (112) du logement jusqu'à libérer l'espace de déformation.

4. Procédé selon la revendication 2 ou 3, dans lequel l'objet (O) est enrobé d'un matériau biocompatible avec la nature de la cornée (1) préalablement à son insertion.

5. Procédé selon l'une quelconque des revendications 2 à 4, dans lequel le matériau est une pierre.

6. Procédé selon l'une quelconque des revendications 2 à 4, dans lequel le matériau est métallique.

7. Procédé selon l'une quelconque des revendications 2 à 4, dans lequel le matériau est une matière plastique.

8. Procédé selon la revendication 1, dans lequel la cavité définit :
au moins un logement (11', 116) de réception du matériau (G) de direction principale approximativement parallèle à la surface de la cornée, le logement débouchant par au moins un premier canal (12') d'introduction d'un outil (26) de dépôt du matériau à l'intérieur du logement, le canal étant obturable par une lèvre déformable (16), ménagée dans l'épaisseur de la cornée (1) ; et
au moins un canal d'évent (17) établissant une deuxième communication entre le logement et la surface de l'oeil.

9. Procédé selon la revendication 8, dans lequel le matériau est un gel (G) ou liquide coloré, non susceptible de diffuser dans la cornée.

10. Système d'insertion d'un objet décoratif (O, G) ou d'un marquage dans la cornée (1) d'un oeil, hors de l'aire pupillaire, comportant :
un laser programmé pour la réalisation d'une cavité conforme à l'une quelconque des revendications 1 à 9 ; et
un matériau (O, G) formant un objet décoratif ou un marquage à loger dans la cavité.
